# EUROPÄISCHE PATENTSCHRIFT

(11) **EP 0 540 857 B1**
(45) Veröffentlichungstag und Bekanntmachung des Hinweises auf die Patenterteilung: **17.09.1997**
(21) Anmeldenummer: 92115515.6
(22) Anmeldetag: 10.09.1992
(51) Int. Cl.: A61K 9/113, B01F 3/08, A61K 7/00, B01F 17/00, B01F 17/42, B01F 17/52

(54) **Verfahren zur Herstellung von stabilen, komplexen Emulsionssystemen des Typs Wasser-Öl-Wasser und Verwendung als kosmetische Präparate**
Process for preparing stable, complex emulsion compositions of the water-oil-water type and use of said compositions as cosmetic preparations
Procédé pour la préparation d'émulsions stables complexes du type eau-huile-eau et application de celles-ci comme produits cosmétiques

(30) Priorität: 07.11.1991 DE 4136699
(43) Veröffentlichungstag der Anmeldung: 12.05.1993
(73) Patentinhaber: LANCASTER GROUP GmbH, 67059 Ludwigshafen (DE)
(72) Erfinder: Ferrero, Louis, F-06000 Nice (FR)
(74) Vertreter: Walter, Wolf-Jürgen

(56) Entgegenhaltungen:
- EP-A- 0 174 377
- FR-A- 236 914
- FR-A- 2 635 458
- US-A- 4 714 566
- JOURNAL OF CONTROLLED RELEASE Bd. 3, 1986, AMSTERDAM Seiten 279 - 290 T.K.LAW ET AL. 'STABILISATION OF W/O/W MULTIBLE EMULSIONS BY INTERFACIAL COMPLEXATION OF MACROMOLECULES AND NONIONIC SURFACTANTS'

## Beschreibung

Die vorliegende Erfindung betrifft ein neuartiges Verfahren zur Herstellung von Mehrfachemulsionen des Typs Wasser-Öl-Wasser(W/O/W), besonders zum Zweck der Verwendung solcher Emulsionen in kosmetischen Produkten.

Die Herstellung solcher Emulsionen ist im Britischen Patent Nr. 1541463(Lion Dentifrice Co.) und in einem Artikel von Florence und Whitehill mit dem Titel " Die Bildung und Stabilität von Mehrfachemulsionen" im International Journal of Pharmaceutics II(1982) S. 277 - 308 beschrieben. Weitere Information kann einem wissenschaftlichen Vortrag mit dem Titel "Les émulsions multiples" , von M. Seiller et al, der beim 2. Weltkongreß für oberflächenaktive Mittel - CESIO, Paris, vom 24.-27.Mai 1988,gehalten wurde, entnommen werden.

Gemäß den genannten Veröffentlichungen werden Wasser/Öl/Wasser-Emulsionen durch Rühren einer Wasserphase und einer Ölphase in Gegenwart eines ersten oberflächenaktiven Mittels unter Bildung einer Wasser/Öl/(Wasser-in-Öl)-Emulsion, und Eingießen der Wasser/Öl-Emulsion in eine Wasserphase, welche ein zweites oberflächenaktives Mittel umfaßt, und durch Rühren der Mischung unter Bildung der Wasser/Öl/Wasser-Mehrfachemulsion hergestellt. Es wird gezeigt, daß die Herstellung von stabilen Mehrfachemulsionen ein schwieriges Problem darstellt.

Aufgabe der Erfindung war es daher, das Problem der Herstellung von solchen komplexen Mehrfachemulsionen, die eine ausreichende Stabilität für die praktische Verwendung aufweisen, zu lösen.

Überraschenderweise hat sich gezeigt, daß es zur Lösung erforderlich ist, in der Zusammensetzung der Primäremulsion(W/O) eine Kombination von nicht-ionischem Emulgator und nicht-ionischem Stabilisator zu verwenden, und daß außerdem die Art der Vereinigung dieser Primäremulsion mit weiteren Mengen einer Wasserphase zwecks Phasenumkehr von Bedeutung ist.

Das erfindungsgemäße Verfahren zur Herstellung von stabilen komplexen Emulsionssystemen des Typs Wasser-Öl-Wasser (W/O/W) ist daher dadurch gekennzeichnet, daß man
a) aus einem Mineralöl und gegebenenfalls zusätzlich einer weiteren Ölkomponente in Form eines Esters einer Fettsäure und/oder eines Fettalkohols sowie Wasser, welches gegebenenfalls eine Komponente mit Hydratisierungs- und/oder Gelierungsaktivität enthält, und in Anwesenheit eines nicht-ionischen polymeren lipophilen Emulgators und eines nicht-ionischen polymeren Stabilisators, jeweils mit nicht zu hohem HLB-Wert, bei erhöhter Temperatur eine Primäremulsion des Typs W/O herstellt,
b) zu dieser Emulsion langsam und unter Rühren ohne Erzeugung von Scherkräften bei Umgebungstemperatur gerade so viel Wasser hinzufügt, welches einen hydrophilen Emulgator mit höherem HLB-Wert enthält, daß die Phasenumkehr eintritt, wobei die gegebenenfalls in Stufe (a) zugesetzten Komponenten mit Hydratisierungs- und/oder Gelierungsaktivität in der inneren wäßrigen Phase der W/O/W-Emulsion eingeschlossen sind, und gegebenenfalls
c) der so gebildeten komplexen W/O/W-Emulsion unter leichtem Rühren bei Umgebungstemperatur eine Komponente mit Hydratisierungs- und/oder Gelierungsaktivität einverleibt.

Die Stufe (b) ist wesentlich, weil diese Umkehr der Zugabe von Primäremulsion und Wasser, verglichen mit der bisherigen Arbeitsweise, es erst ermöglicht, tatsächlich stabile Mehrfachemulsionen herzustellen, wie nachstehend anhand von Beispielen und Versuchen noch erläutert wird.

Die Bildung der gewünschten Mehrfachemulsion läßt sich durch Überwachen des Verfahrens mittels Leitfähigkeitsmessung bis zum Erreichen des Emulsions-Umkehrungspunktes feststellen.

Der Vorteil des Umkehrzugabeverfahrens besteht außer der Stabilität der W/O/W-Emulsionen darin, daß nur ein einziger Emulgier-Mischer für die Herstellung der Mehrfachemulsion benötigt wird.

Durch die Maßnahme der Zugabe von Verdickungs- oder Geliermitteln in Stufe (a) bzw. durch die entsprechende abschließende Maßnahme gemäß Stufe (c) kann die Viskosität der Mehrfachemulsion modifiziert werden, was für die Verwendung als kosmetisches Präparat, z.B. in Form einer Flüssigmilch oder einer Creme, von Bedeutung ist.

Als weitere Ölkomponente wird in Stufe (a) zweckmäßig ein Pflanzenöl , insbesondere Borretschöl verwendet. Borretschöl enthält Triglyceride von γ-Linolensäure und wird aus dem Samen der Borretschpflanze erhalten.

Es können aber auch andere Öle, wie tierische Öle, eingesetzt werden.

Die Verfahrensstufe(a) wird zweckmäßig bei erhöhter Temperatur bis 70°C durchgeführt , um eine gute Vermischung aller festen und/oder flüssigen Komponenten sicherzustellen.

Der in der Verfahrensstufe (a) eingesetzte lipophile Emulgator hat vorzugsweise einen HLB-Wert(hydrophilic liphophilic balance) im Bereich von 6 bis 10.

Insbesondere eignet sich hierfür ein Methoxy-Polyäthylenglykol-Dodecylglykol-Copolymer ,z.B. ein im Handel erhältliches polymeres Produkt des Typs Methoxy-Polyäthylenglykol(22)-Dodecylglykol, welches der folgenden Formel entspricht:

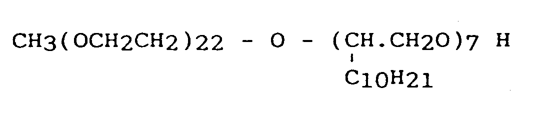

Ein solches Copolymer hat z.B. ein Durchschnittsmolekulargewicht von ca. 1800.

Bei dem in Verfahrensstufe(a) mitverwendeten Stabilisator handelt es sich gleichfalls um ein polymeres Produkt, dessen HLB-Wert zweckmäßig ebenfalls im Bereich von 6 bis 10 liegt. Sehr geeignet für diesen Zweck sind Copolymere des gleichen Typs wie der Emulgator, z.B. ein Polyäthylenglykol-Dodecylglykol-Copolymer. Es kann dabei geeigneter Weise ein Handelsprodukt mit einem Durchschnittsmolekulargewicht im Bereich von 2300 bis 4000 eingesetzt werden, z.B. ein Polymer der nachstehenden Formel

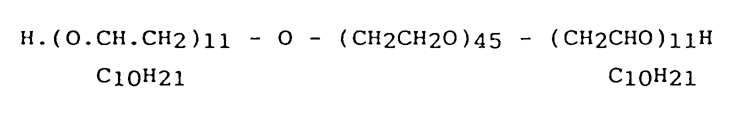

In der Verfahrensstufe (b) wird in der wäßrigen Phase ein hydrophiler Emulgator mitverwendet, der zweckmäßig einen HLB-Wert im Bereich von 12 bis 25 und insbesondere von 15 bis 22 aufweist. Sehr geeignet sind hierfür im Handel erhältliche Äthylenoxid-Propylenoxid-Blockcopolymerisate mit Durchschnittsmolekulargewichten zwischen 10000 und 15000, z.B ein Copolymer der nachstehenden Formel

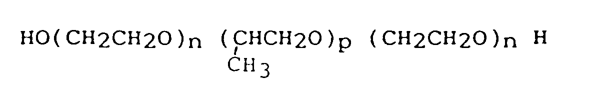

Ein weiteres geeignetes Handelsprodukt ist ein Methoxy-Polyäthylenglykol(17)-Dodecylglykol-Copolymer mit einem HLB-Wert von 15.

Der Ölphase kann man vor oder nach Bildung der Primäremulsion in Verfahrensstufe (a) eine oder mehrere öllösliche Aktivkomponenten zusetzen, wie unverseifbare Bestandteile von pflanzlichen Ölen, z.B. von Zea Mays, Vitamine, z.B. Dl-α-Tocopherolacetat, und UV-Filter, wie Octylmethoxycinnamat.

Die Wasser-in-Öl-Emulsion kann außerdem Hydratisier- oder Geliermittel, wie z.B. Polyglycerylmethacrylat und Propylenglykol(Handelsprodukt LUBRAJEL CG), Natrium-Hyaluronatgel oder Aloe-Vera-Gel enthalten. Sie kann ferner Cerebroside, Cholesterin oder Phospholipide enthalten, die als zusätzliche Stabilisatoren wirken können.

Als Viskositätsmodifikatoren oder Geliermittel für die Mehrfachemulsion seien Polyglycerylmethacrylat und Xanthangummi erwähnt.

Eine Mehrfachemulsion, die unter Verwendung der genannten Stoffe erfindungsgemäß hergestellt wird, fühlt sich beim Auftragen auf die Haut besonders angenehm an.

Zur Veranschaulichung des Vorteils des erfindungsgemäßen Verfahrens dienen die nachstehenden Beispiele:

### Beispiel 1:

### Zusammensetzung der Primäremulsion (%)

| | Emulsion 1 | Emulsion 2 | Emulsion 3 | Emulsion 4 |
|---|---|---|---|---|
| Mineralöl | 26 | 26 | 26 | 26 |
| Elfacos ^{(R)} E 200^{x)} (Emulgator) | 4 | 0 | 2 | 1 |
| Elfacos^{(R)} ST9^{xx)} (Stabilisator) | 0 | 4 | 2 | 3 |
| H₂O | 66 | 66 | 66 | 66 |
| NaCl | 4 | 4 | 4 | 4 |

| | | | | |
|---|---|---|---|---|
| x) Im Handel erhältlicher nicht-ionischer polymerer Emulgator des Typs Methoxy-Polyäthylenglykol(22)-Dodecylglykol-Copolymer(HLB-Wert ca. 8,5) | | | | |
| xx) Im Handel erhältlicher nicht-ionischer polymerer Stabilisator des Typs Polyäthylenglykol(40)-Dodecylglykolcopolymer mit einem HLB-Wert von 6,5. | | | | |

Verfahren: für 300 g Primäremulsion: die Ölphase, zusammengesetzt aus Mineralöl, Emulgator und Stabilisator, wird auf 70°C erwärmt.
Natriumchlorid und Wasser werden auf 70°C erhitzt. Diese Wasserphase wird zwecks Erhalt einer W/O-Emulsion zur Ölphase zugegeben, welche bei hoher Geschwindigkeit mit einem Homogenisierungsmischer bis zur Abkühlung auf Raumtemperatur gerührt wird.

### Zusammensetzung der Wasserphase(%)

| | |
|---|---|
| Synperonic^{(R)} PE/F 127* | 5 |
| Wasser | 94,6 |
| Glydant** | 0,4 |

| | |
|---|---|
| *)Im Handel erhältlicher hydrophiler Emulgator in Form eines Äthylenoxid-Propylenoxid-Blockcopolymers mit hohem MG(ca. 12000) und einem HLB-Wert von 22. | |
| **)In kosmetischen Produkten eingesetztes Konservierungsmittel | |

Verfahren: Die Auflösung dieser Stoffe in Wasser erfolgt bei Raumtemperatur unter Rühren, wobei das Auftreten von Scherkräften vermieden wird.

### Umkehrung der Primäremulsion

Verfahren: Die Wasserphase wird langsam unter leichtem Rühren bei Raumtemperatur zur Primäremulsion zugegeben.

Die Phasenumkehrung wird optisch sichtbar. Sie wird mittels eines Leitfähigkeitsmessers überprüft. Die folgende Zusammensetzung der Endemulsion in % wird nach der Phasenumkehrung erhalten:

| | Emulsion 1 | Emulsion 2 | Emulsion 3 | Emulsion 4 |
|---|---|---|---|---|
| % Primäremulsion | 95 | 80 | 87 | 83 |
| % Wasserphase | 5 | 20 | 13 | 17 |
| Viskosität nach 3 Monaten, mPa·s (cps) | 5661 | 7492 | 7326 | 11600 |
| Leitfähigkeit nach 3 Monaten, mS/cm | 2,82 | 1,59 | 1,72 | 1,32 |
| Struktur dieser Emulsion | Mehrfach-W/O/W | desgl.mit großen Kügelchen | desgl. | desgl. |
| Stabilität dieser Emulsion nach 1 Jahr | schlecht: Entmischung | gut | gut | gut |

Die W/O/W-Struktur wird durch mikroskopische Prüfung festgestellt.

Bemerkung: Der Haupteffekt der Mitverwendung von Elfacos^{(R)} ST9(Stabilisator der W/O-Primäremulsion) besteht darin, den Gehalt an Wasserphase so zu erhöhen, daß der für das Erreichen des Phasenumkehrpunktes erforderliche Gehalt vorliegt. Die erhaltene Mehrfachemulsion ist stabiler bei Anwesenheit des Stabilisators.

### Beispiel 2

Struktur der Primäremulsion: Siehe Emulsion 4.
Struktur der Wasserphase: wie vorstehend beschrieben.

### Umkehrung der Primäremulsion

Folgende Ergebnisse werden erhalten:

| | Versuch 1 | Versuch 2 | Versuch 3 |
|---|---|---|---|
| Bedingungen der Phaseninversion | Zugabe der Wasserphase gleich nach Umkehrung gestoppt | Überschuß an Wasserphase nach Umkehrung | hoher Überschuß nach Umkehrung |
| % Primäremulsion | 82 | 73 | 64 |
| % Wasserphase | 18 | 27 | 36 |
| Viskosität nach 3 Monaten, mPa·s (cps) | 10000 | 7000 | 4000 |
| Leitfähigkeit nach 3 Monaten,mS/cm | 1,45 | 2,42 | 4,29 |
| Struktur dieser Emulsion | Mehrfach W/O/W-Emulsion | desgl. | nicht klar |
| Stabilität der Emulsion | gut | Entmischung | starke Entmischung |

Bemerkung: Die beste W/O/W-Stabilität wird erhalten, wenn die Zugabe von Wasserphase gleich nach der Phasenumkehrung gestoppt wird.

Für die weiteren Beispiele wird die Zusammensetzung der Wasserphase modifiziert: Lubrajel^{(R)} CG(Polyglycerylmethacrylat und Propylenglykol) wird der inneren wäßrigen Phase der Primäremulsion als Komponente mit Hydratisierungs- und Gelierungaktivität zugesetzt. Natriumchlorid muß wegen Inkompatibilität mit diesem Geliermittel fortgelassen werden.

### Beispiel 3

### Zusammensetzung der Primäremulsion(%)

| | Emulsion 5 | Emulsion 6 |
|---|---|---|
| Mineralöl | 26 | 16 |
| Borretschöl | 0 | 4 |
| Octylmethoxycinnamat | 0 | 4 |
| Elfacos^{(R)} E200 | 1 | 1 |
| Elfacos^{(R)} ST9 | 3 | 3 |
| H₂O | 59,5 | 59,5 |
| Lubrajel^{(R)} CG | 10 | 10 |
| DL-alpha-Tocopherolacetat | 0 | 2 |
| Phenonip* | 0,5 | 0,5 |

| | | |
|---|---|---|
| * In kosmetischen Produkten verwendetes Konservierungsmittel: Phenoxyäthanol und (Methyl-Äthyl-Propyl-Butyl)paraben. | | |

Verfahren: Für 300 g Primäremulsion, wie vorstehend für das Verfahren von Beispiel 1 beschrieben, mit Ausnahme der Zugabe von DL-alpha-Tocopherolacetat, welches nach dem Emulgieren bei ca. 40°C zugegeben wird. Das Lubrajel^{(R)}CG wird vorher in der Wasserphase gelöst.

Wasserphasenzusammensetzung: wie in Beispiel 1 beschrieben.

Auswertung der Ergebnisse bei der Herstellung von Mehrfachemulsionen:

### 2 Methoden werden angewandt:

1.Technik der Dispersion der W/O-Primäremulsion in der Wasserphase nach dem von Seiller beschriebenen Verfahren (loc.cit.). Die Zugabe der Primäremulsion wird nach dem Verdicken der Dispersion gestoppt.
2. Technik gemäß der Erfindung, wie vorstehend in Beispiel 1 und 2 beschrieben: Phasenumkehrung in 2 Stufen.

| | Emulsion 5 | | Emulsion 6 | |
|---|---|---|---|---|
| Technik der Umwandlung von W/O | Phasenumkehr | W/O-Dispersion | Phasenumkehr | W/O-Dispersion |
| % Primäremulsion | 86 | 85 | 59 | 75 |
| % Wasserphase' | 14 | 15 | 41 | 25 |
| Viskosität nach 3 Monaten, mPa·s (cps) | flüssig | flüssig | 11200 | flüssig |
| Leitfähigkeit nach 3 Monaten,µS/cm | 35 | 30 | 16 | 20 |
| Struktur der Emulsion | nicht mehrfach, einfache Kügelchen | nicht mehrfach, einfache Kügelchen | mehrfach-W/O/W | nicht mehrfach,einfache Kügelchen |
| Stabilität der Emulsion nach 3 Monaten | Entmischung | Entmischung | gut | Entmischung |

Bemerkung: Im Fall der Emulsion Nr.5 gibt es keine Möglichkeit, eine Mehrfachemulsion mit Lubrajel CG und ohne Natriumchlorid zu erhalten.Im Gegensatz hierzu macht es bei Emulsion Nr.6 die Mitverwendung von öligen Komponenten, die polare Gruppen enthalten, möglich, nach dem Phasenumkehrverfahren eine komplexe Emulsion herzustellen.Emulsion Nr. 6 enthält als polare Komponenten Borretschöl, Octylmethoxycinnamat und DL-α-Tocopherolacetat.

### Beispiel 4:

Mitverwendung weiterer aktiver Bestandteile in der Primäremulsion .

### Zusammensetzung der Primäremulsion(%)

| | Emulsion 7 | Emulsion 8 | Emulsion 9 | Emulsion 10 |
|---|---|---|---|---|
| Mineralöl | 15,9 | 15,5 | 14,9 | 14,9 |
| Borretschöl | 4 | 4 | 4 | 4 |
| Octylmethoxycinnamat | 4 | 4 | 4 | 4 |
| Cholesterin | 0,1 | 0 | 0,1 | 0,1 |
| Nicht verseifbare | 0 | 0,5 | 0,5 | 0,5 |
| Substanz von Zea Mays Ceramide^{x)} | 0 | 0 | 0,5 | 0,5 |
| Elfacos^{(R)} E200 | 1 | 1 | 1 | 1 |
| Elfacos^{(R)} ST9 | 3 | 3 | 3 | 1,5 |
| Wasser | 59,5 | 59,5 | 59,5 | 61 |
| Lubrajel CG | 10 | 10 | 10 | 10 |
| Tocopherolacetat | 2 | 2 | 2 | 2 |
| Phenonip | 0,5 | 0,5 | 0,5 | 0.5 |

| | | | | |
|---|---|---|---|---|
| x) Dieser Komplex-Zusatzstoff besteht aus einer Mischung von Glycosphinglolipiden, Phospholipiden und Cholesterin. | | | | |

Verfahren: wie das Verfahren von Beispiel 3.
Wasserphasenzusammensetzung: wie bei Beispiel 1

Umwandlung der W/O-Primäremulsion in eine komplexe Emulsion durch Phasenumkehrung.
Folgende Ergebnisse werden erhalten:

| | Emulsion 7 | Emulsion 8 | Emulsion 9 | Emulsion 10 |
|---|---|---|---|---|
| % Primäremulsion | 60 | 66 | 33 | 67 |
| % Wasserphase | 40 | 34 | 67 | 33 |
| Viskosität nach 3 Monaten, mPa·s (cps) | 25200 | 21000 | 12000 | 15000 |
| Leitfähigkeit nach 3 Monaten,µS/cm | 5,8 | 8,0 | 5,0 | 18 |
| Struktur der Emulsion | mehrfach W/O/W | mehrfach W/O/W | mehrfach, aber kaum in Wasser dispergierbar | mehrfach W/O/W |
| Stabilität der Emulsion nach 3 Monaten | gut | gut | gut | gut |

### Bemerkung:

Cholesterin und die nicht verseifbare Substanz von Zea Mays haben keine große Wirkung auf die Volumenfraktion der Wasserphase, die zum Erreichen des Phasenumkehrpunkts(bei den untersuchten Konzentrationen) notwendig ist.

Dies gilt nicht für Ceramide, welche dieses Volumen erheblich vergrößern. Man kann davon ausgehen, daß dieses Material als lipophiler Emulgator(siehe Emulsion 9) wirkt.

Dank der Möglichkeit, die Stabilisatorkonzentration zu beeinflussen(Elfacos ^{(R)}ST9: Abnahme von 3% auf 1,5%), läßt sich eine gute W/O/W-Emulsion mit einem normalen Gehalt an Wasserphase(siehe Emulsion 10) erzielen.

### Beispiel 5:

### W/O/W-Mehrfachemulsion-Gelierung gemäß Verfahrensstufe (c)

Zusammensetzung der Primäremulsion: siehe Emulsion 10 in Beispiel 4.

Umwandlung dieser W/O-Primäremulsion in die Komplexemulsion durch Phasenumkehrung: siehe die Ergebnisse, die zuvor mit der Emulsion in Beispiel 4 erhalten worden sind.

Abschließendes Gelieren der Mehrfachemulsion 10:

| | |
|---|---|
| Anteil an W/O/W-Emulsion 10 : | 95% |
| Anteil an Lubrajel^{(R)} CG: | 5% |

Lubrajel^{(R)} CG wird unter Rühren, wobei das Auftreten von Scherkräften vermieden wird, langsam zu der komplexen Mehrfachemulsion zugegeben, bis dieser Bestandteil vollständig dispergiert ist.

Zunächst nimmt die Viskosität ab, aber nach 24-stündigem Stehen erscheint die Emulsion gut geliert und verbleibt in diesem Zustand,wie es aus der Tabelle ersichtlich ist.

| | Emulsion 10 vor dem Gelieren | Emulsion 10 nach dem Gelieren |
|---|---|---|
| Viskosität nach 3 Monaten, mPa·s (cps) | 15000 | 28 400 |
| Leitfähigkeit nach 3 Monaten,µS/cm | 18 | 15 |
| Struktur der Emulsion (unter dem Mikroskop) | Mehrfach W/O/W | Mehrfach W/O/W |
| Emulsionsstabilität nach 3 Monaten | gut | gut |

Durch Zusatz von Parfümstoffen und gegebenenfalls weiteren in der Kosmetik üblichen Wirkstoffen können aus den erfindungsgemäß hergestellten stabilen Mehrfachemulsionen kosmetische Präparate erhalten werden, die zu beliebiger Konsistenz verdickt sein können. Sie lassen sich infolge ihrer günstigen thixotropen Eigenschaften gut auf der Haut verteilen und haben eine gute Hautverträglichkeit. Emulsion Nr.10 hat nach dem Gelieren ausgezeichnete kosmetische Eigenschaften und bewirkt eine dauerhafte Feuchtigkeitsretention auf der Haut.

## Patentansprüche

1. Verfahren zur Herstellung von stabilen komplexen Emulsionssystemen des Typs Wasser-Öl-Wasser (W/O/W),
**dadurch gekennzeichnet, daß** man
a) aus einem Mineralöl und gegebenenfalls zusätzlich einer weiteren Ölkomponente in Form eines Esters einer Fettsäure und/oder eines Fettalkohols sowie Wasser, welches gegebenenfalls eine Komponente mit Hydratisierungs- und/oder Gelierungsaktivität enthält, und in Anwesenheit eines nicht-ionischen polymeren lipophilen Emulgators und eines nicht-ionischen polymeren Stabilisators jeweils mit einem HLB-Wert im Bereich von 6 bis 10, bei erhöhter Temperatur eine Primäremulsion des Typs W/O herstellt,
b) zu dieser Emulsion langsam und unter Rühren ohne Erzeugung von Scherkräften bei Umgebungstemperatur gerade so viel Wasser hinzufügt, welches einen hydrophilen Emulgator mit höherem HLB-Wert im Bereich von 12-25 enthält, daß die Phasenumkehr eintritt, wobei die gegebenenfalls in Stufe (a) eingesetzten Komponenten mit Hydratisierungs- und/oder Gelierungsaktivität in der inneren wäßrigen Phase der W/O/W-Emulsion eingeschlossen sind, und gegebenenfalls
c) der so gebildeten komplexen W/O/W-Emulsion unter leichtem Rühren bei Umgebungstemperatur eine Komponente mit Hydratisierungs- und/oder Gelierungsaktivität einverleibt.

2. Verfahren nach Anspruch 1, dadurch gekennzeichnet, daß man als weitere Ölkomponente ein Pflanzenöl, insbesondere Borretschöl verwendet.

3. Verfahren nach Anspruch 1, dadurch gekennzeichnet, daß man als weitere Ölkomponente ein tierisches Öl verwendet.

4. Verfahren nach Anspruch 1 bis 3, dadurch gekennzeichnet, daß man in Verfahrensstufe (a) bei Temperaturen bis 70° C arbeitet.

5. Verfahren nach Anspruch 1, dadurch gekennzeichnet, daß der lipophile Emulgator ein Methoxy-Polyethylenglykol-Dodecylglykol-Copolymer ist.

6. Verfahren nach Anspruch 5, dadurch gekennzeichnet, daß der lipophile Emulgator ein Durchschnittsmoleklargewicht von 1800 aufweist.

7. Verfahren nach Anspruch 1, dadurch gekennzeichnet, daß der Stabilisator ein Polyethylenglykol-Dodecylglykol-Copolymer ist.

8. Verfahren nach Anspruch 7, dadurch gekennzeichnet, daß der Stabilisator ein Durchschnittsmolekulargewicht im Bereich von 2300 bis 4000 hat.

9. Verfahren nach Anspruch 1 bis 8, dadurch gekennzeichnet, daß man in Verfahrensstufe (b) einen hydrophilen Emulgator mit einem HLB-Wert im Bereich von 12 bis 25, vorzugsweise im Bereich von 15 bis 22 verwendet.

10. Verfahren nach Anspruch 9, dadurch gekennzeichnet, daß man als hydrophilen Emulgator ein Ethylenoxid-Propylenoxid-Blockkcopolymer verwendet.

11. Verfahren nach Anspruch 10, dadurch gekennzeichnet, daß der hydrophile Emulgator ein Durchschnittsmolekulargewicht im Bereich von 10000 bis 15000 aufweist.

12. Verfahren nach Anspruch 1 bis 11, dadurch gekennzeichnet, daß man der Ölphase vor oder nach der Emulsionsbildung eine oder mehrere öllösliche Aktivkomponenten einverleibt.

13. Verfahren nach Anspruch 12, dadurch gekennzeichnet, daß man als UV-Filter wirkende Aktivkomponenten oder Vitamine mitverwendet.

14. Verfahren nach Anspruch 12 und 13, dadurch gekennzeichnet, daß man als UV-Filter die Verbindung Octylmethoxycinnamat und als Vitamin den Vitamin E-Ester (DL-α-Tokopherolacetat) mitverwendet.

15. Verfahren nach Anspruch 1 bis 14, dadurch gekennzeichnet, daß man in der wäßrigen Phase vor oder nach der Bildung der Mehrfachemulsion als Komponenten mit Hydratisierungsaktivität und Gelierungswirkung Propylenglykol und/oder ein Polyglycerylmethacylat mitverwendet.

16. Verfahren nach Anspruch 1 bis 15, dadurch gekennzeichnet, daß man bei der Herstellung der Primäremulsion weitere Aktivkomponenten mitverwendet, insbesondere unverseifbare Bestandteile von pflanzlichen Ölen, wie von Zea Mays, Na-Hylaronatgel, Aloe-Vera-Gel, Xanthat, Cerebroside, Cholesterin und/oder Phospholipide.

## Claims

1. The process according to the invention for the preparation of stable complex emulsion systems of the water-oil-water (W/O/W) type is thus characterised in that
a) a primary emulsion of the W/O type is prepared at elevated temperature from a mineral oil and, if necessary, additionally a further oil component in the form of an ester of a fatty acid and/or in the form of a fatty alcohol, and water which, if necessary, contains a component having hydrating and/or gelling activity, and in the presence of a non-ionic polymeric lipophilic emulsifier and a non-ionic polymeric stabiliser, both having an HLB value in the range of 6 to 10,
b) just enough water containing a hydrophilic emulsifier having a higher HLB value in the range of 12 to 25 is added to this emulsion, slowly and with stirring without generating shear forces, at ambient temperature, so that phase inversion occurs, the components having hydrating and/or gelling activity which were added if necessary in step (a) being included in the inner aqueous phase of the W/O/W emulsion, and, if necessary,
c) a component having hydrating and/or gelling activity is incorporated into the complex W/O/W emulsion thus formed, with gentle stirring at ambient temperature.

2. Process according to Claim 1, characterised in that the further oil component used is a vegetable oil, in particular oil of borage.

3. Process according to Claim 1, characterised in that the additional oil component used is an animal oil.

4. Process according to Claims 1 to 3, characterised in that process step (a) is carried out at temperatures of up to 70°C.

5. Process according to Claim 1, characterised in that the lipophilic emulsifier is a methoxypolyethylene glycol-dodecyl glycol copolymer.

6. Process according to Claims 5, characterised in that the lipophilic emulsifier has an average molecular weight of 1,800.

7. Process according to Claim 1, characterised in that the stabiliser is a polyethylene glycol-dodecyl glycol copolymer.

8. Process according to Claim 7, characterised in that the stabiliser has an average molecular weight in the range from 2,300 to 4,000.

9. Process according to Claims 1 to 8, characterised in that a hydrophilic emulsifier with an HLB value in the range from 12 to 25, preferably in the range from 15 to 22, is used in process step (b).

10. Process according to Claim 9, characterised in that the hydrophilic emulsifier used is an ethylene oxide-propylene oxide block copolymer.

11. Process according to Claim 10, characterised in that the hydrophilic emulsifier has an average molecular weight in the range from 10,000 to 15,000.

12. Process according to Claims 1 to 11, characterised in that one or more oil-soluble active components are incorporated into the oil phase before or after the formation of the emulsion.

13. Process according to Claim 12, characterised in that active components which act as UV filters or vitamins are used in addition.

14. Process according to Claims 12 and 13, characterised in that the UV filter used is the compound octyl methoxycinnamate and the vitamin used is vitamin E ester (dl-α-tocopherol acetate).

15. Process according to Claim 1 to 14, characterised in that the components having a hydrating activity and gelling effect which are used in the aqueous phase before or after the formation of the multiple emulsion are propylene glycol and/or a polyglyceryl methacrylate.

16. Process according to Claims 1 to 15, characterised in that in the preparation of the primary emulsion further active components are used, in particular unsaponifiable components of vegetable oils, such as from Zea Mays, Na hyaluronate gel, Aloe vera gel, xanthate, cerebrosides, cholesterol and/or phospholipids.

## Revendications

1. Procédé pour la préparation d'émulsions complexes stables du type eau-huile-eau (W/O/W)
**caractérisé en ce que**
a) on prépare une émulsion primaire du type W/O à partir d'une huile minérale à laquelle on a éventuellement ajouté un autre constituant huileux sous la forme d'un ester d'un acide gras et/ou d'un alcool gras, et d'eau comprenant éventuellement un constituant actif pour l'hydratation et/ou la gélification, et ce à haute température et en présence d'un émulsifiant polymère non-ionique lipophile et d'un stabilisant polymère non-ionique présentant tous les deux un HLB (équilibre hydrophile-lipophile) pas trop élevé,
b) à température ambiante, on ajoute à cette émulsion, lentement et en mélangeant sans produire d'efforts de cisaillement, la quantité exacte d'eau nécessaire pour provoquer une inversion de phase, cette eau contenant un émulsifiant hydrophile à HLB plus élevé, les composants actifs pour l'hydratation et/ou la gélification éventuellement ajoutés lors de l'étape (a) étant inclus dans la phase aqueuse interne de l'émulsion W/O/W, et éventuellement,
c) en ce que l'émulsion W/O/W complexe ainsi préparée incorpore un composant actif pour l'hydratation et/ou la gélification, et ce pendant un mélange léger à température ambiante.

2. Procédé selon la revendication 1 caractérisé en ce qu'on peut utiliser une huile végétale, notamment l'huile de bourrache, comme autre composant huileux.

3. Procédé selon la revendication 1 caractérisé en ce qu'on peut utiliser une huile animale comme autre composant huileux.

4. Procédé selon les revendications 1 à 3 caractérisé en ce qu'on travaille à des températures allant jusqu'à 70°C dans l'étape (a) du processus.

5. Procédé selon la revendication 1 caractérisé en ce que l'émulsifiant lipophile est un un copolymère de méthoxypolyéthylèneglycol-dodécylglycol.

6. Procédé selon la revendication 5 caractérisé en ce que le poids moléculaire moyen de l'émulsifiant lipophile est de 1800.

7. Procédé selon la revendication 1 caractérisé en ce que le stabilisant est un un copolymère de polyéthylèneglycol-dodécylglycol.

8. Procédé selon la revendication 5 caractérisé en ce que le poids moléculaire moyen du stabilisant est compris entre 2300 et 4000.

9. Procédé selon les revendications 1 à 8 caractérisé en ce que dans l'étape (b) du procédé, on emploie un émulsifiant lipophile ayant un HLB compris entre 12 et 25, de préférence entre 15 et 22.

10. Procédé selon la revendication 9 caractérisé en ce qu'on utilise un copolymérisat séquencé oxyde d'éthylène - oxyde de propylène comme émulsifiant hydrophile.

11. Procédé selon la revendication 10 caractérisé en ce que le poids moléculaire moyen de l'émulsifiant hydrophile est compris entre 10000 et 15000.

12. Procédé selon les revendications 1 à 11 caractérisé en ce qu'on incorpore à la phase huileuse un ou plusieurs composants actifs solubles dans l'huile, et ce avant ou après la formation de l'émulsion.

13. Procédé selon la revendication 12 caractérisé en ce qu'on utilise simultanément des composants actifs agissant comme des filtres UV ou des vitamines.

14. Procédé selon les revendications 12 et 13 caractérisé en ce qu'on emploie également le composé méthoxycinnamate d'octyle comme filtre UV et l'ester de vitamine E (DL acétate d'alpha tocophéryle) comme vitamine.

15. Procédé selon les revendications 1 à 14 caractérisé en ce qu'on emploie également dans la phase aqueuse du polypropylèneglycol et/ou du méthycrylate polyglycérylique comme composants ayant une activité hydratante et un effet gélifiant, et ce avant ou après la formation de l'émulsion multiple.

16. Procédé selon les revendications 1 à 15 caractérisé en ce qu'on utilise également d'autres composans actifs lors de la préparation de l'émulsion primaire, notamment des constituants insaponifiables d'huiles végétales telles que les substances de Zea Mays, le gel d'hylaronate de sodium, le gel d'aloa vera, le xanthate, la cérébroside, le cholestérol et/ou les phospholipides.
